# EUROPEAN PATENT APPLICATION

(11) **EP 3 025 629 A1**
(43) Date of publication of application: **01.06.2016**
(21) Application number: 14828873.1
(22) Date of filing: 16.07.2014
(51) Int. Cl.: A61B 1/00, A61B 17/28, A61B 17/32, A61B 18/14

(54) **MEDICAL DEVICE AND MEDICAL SYSTEM**

(30) Priority: 26.07.2013 JP 2013155886
(71) Applicant: Olympus Corporation, Shibuya-ku Tokyo 151-0072 (JP)
(72) Inventor: OGAWA, Ryohei, Tokyo 151-0072 (JP); KISHI, Kosuke, Tokyo 151-0072 (JP)
(74) Representative: Schicker, Silvia
(86) International application number: PCT/JP2014/068871
(87) International publication number: WO 2015/012163

(57) **Abstract**

Provided is a medical apparatus (1) including a treatment tool (2) having a distal-end treatment section (22), a joint section (23), and a flexible elongated body section (21) in this order from the distal end; a driver (3) that is connected to the base end of the body section (21) and that supplies a driving force to the joint section (23); an operation input unit (4) that is a separate unit from the treatment tool (2) and the driver (3) and that has a main body (41) holdable with one hand of an operator and an operable member (42) disposed at a position where it is operable using a finger of the one hand holding the main body (41); and a controller (5) that controls the driver (3) in accordance with an operation command input to the operation input unit (4).

## Description

### {Technical Field}

The present invention relates to medical apparatuses and medical systems.

### {Background Art}

A known flexible treatment tool in the related art that is used by being inserted into a channel of an endoscope is equipped with an operation input unit at the base end of the treatment tool. The operation input unit is for electrically manipulating joints of the treatment tool (for example, see Patent Literatures PTL 1 and PTL 2 below). This operation input unit is provided with a joystick to be used by an operator for instructing the movement directions of the joints and a driver that drives the joints based on the instructions input to the joystick.

### {Citation List}

### {Patent Literature}

{PTL 1} Japanese Unexamined Patent Application, Publication No. 2009-101077
{PTL 2} Japanese Unexamined Patent Application, Publication No. Hei 5-192348

### {Summary of Invention}

### {Technical Problem}

However, because the operation input unit containing the driver, such as an actuator, is heavy, as in Patent Literatures PTL 1 and PTL 2, there are problems in terms of operability in various scenarios during use. For example, since the operator has to keep holding the heavy operation input unit, there is a disadvantage in that the level of fatigue of the operator is high.

Furthermore, in normal use, the operator holds an operable unit of the endoscope with his/her left hand and the treatment tool with his/her right hand, and performs both advancing-retracting and twisting operations of the treatment tool and manipulation of the joints with the right hand alone. In this case, if the advancing-retracting and twisting operations of the treatment tool are performed while holding the operation input unit at a position distant from an inlet of the channel, the treatment tool may buckle or elastically deform at an intermediate location. This may be problematic in that it makes it difficult to transmit advancing-retracting and twisting operations performed close-at-hand to the distal end of the treatment tool.

The present invention has been made in view of the circumstances described above and an object thereof is to provide a medical apparatus and a medical system with high operability and that allow advancing-retracting and twisting operations of a treatment tool having joints and manipulation of the joints to be both performed with ease by using only one hand.

### {Solution to Problem}

In order to achieve the aforementioned object, the present invention provides the following solutions.

A first aspect of the present invention provides a medical apparatus including a treatment tool having an elongated body section that has flexibility, a distal-end treatment section that is located at a distal end of the body section, and a joint section that couples the body section and the distal-end treatment section to each other; a driver that is connected to a base end of the body section and that supplies a driving force to the joint section for actuating the joint section; an operation input unit having a main body and an operable member and being a separate unit from the treatment tool and the driver, the main body having a size and shape that can be held with one hand of an operator, the operable member being disposed at a position where the operable member is operable using a finger of the one hand holding the main body and receiving an operation command for manipulating the joint section; and a controller that controls the driver in accordance with the operation command input to the operation input unit.

According to the first aspect of the present invention, when the operator holds the main body of the operation input unit with one of his/her hands and operates the operable member using any of the fingers, the controller, upon receiving this operation command, controls the driver, and the driver causes the joint section to move in accordance with the operation command. Thus, the operator can change the orientation of the distal-end treatment section. Moreover, the operator holds the body section with the remaining finger or fingers of the hand holding the main body and performs advancing-retracting and twisting operations on this body section so as to cause the distal-end treatment section to advance and recede and to rotate.

In this case, since the operation input unit and the driver, which contains a heavy component, such as a motor, are separate units, the positions of the two can be selected independently of each other. Specifically, the driver may be supported by, for example, the floor, and the operator may support the operation input unit alone, which is light in weight. Thus, the advancing-retracting and twisting operations of the treatment tool and the manipulation of the joints can both be performed with ease by using only one hand, thereby achieving high operability.

In the first aspect, the operation input unit may be connectable to and disconnectable from the controller.

Accordingly, the operation input unit is separated from the controller, which contains components vulnerable to a sterilization process, so that the operation input unit alone can be sterilized.

In the first aspect, the body section may include a grip portion having a non-circular cross-sectional shape and disposed in an area located outside a channel in a medical device in a state where the body section is inserted in the channel.

Accordingly, the operator can stably hold the grip portion. In particular, rotational torque can be readily applied to the body section when the grip portion is twisted.

In the first aspect, the operation input unit may include a gripping member that grips the body section by sandwiching the body section together with the main body in a radial direction of the body section.

Accordingly, the load on the finger or fingers can be reduced, as compared with a case where the operator holds the body section with his/her finger or fingers. Moreover, in a case where the grip portion having the non-circular cross-sectional shape is gripped by the gripping member, the body section can be stably gripped by the gripping member.

In the first aspect, the medical apparatus may further include a rotatable support mechanism that rotatably supports a base end portion of the body section.

Accordingly, when the operator twists the body section, torsion occurs toward the base end of the body section relative to the position held by the operator, so that rotational torque is generated at the base end portion of the body section. The base end portion of the body section rotates in accordance with this rotational torque so as to cancel out the torsion.

In the first aspect, the joint section may include a plurality of joints that are rotatable about axes in directions different from each other. The controller may control the driver such that each joint set in correspondence with a direction of the operation command input to the operable member is rotated in accordance with the direction of the operation command. Moreover, the medical apparatus may further include a correspondence changing unit that changes a correspondence relationship in the controller between the direction of the operation command and each joint.

Accordingly, when the distal-end treatment section is to be manipulated while observing an image of the distal-end treatment section with, for example, an endoscope, since the rotational directions of the joints within the image vary depending on the orientation of the distal-end treatment section in the circumferential direction thereof, the correspondence relationship between the operational direction of the operable member and the movement direction of the distal-end treatment section within the image also changes. In this case, the correspondence relationship between the direction of the operation command and each joint is changed by the correspondence changing unit so that the operational direction of the operable member and the movement direction of the distal-end treatment section within the image correspond with each other, thereby constantly ensuring that the distal-end treatment section can be manipulated intuitively.

In the first aspect, the operation input unit may include a treatment tool identifier that identifies the treatment tool serving as a manipulation target to be manipulated with the operation input unit, and the controller may control the driver in accordance with the treatment tool identified by the treatment tool identifier. In this case, the treatment tool may include two or more of the treatment tools.

Accordingly, in a case where a plurality of treatment tools are to be used in an interchangeable manner, the treatment tool currently used by the operator is identified by the treatment tool identifier so that the joint section of any one of the treatment tools can be properly actuated.

In the first aspect, in a case where the controller cannot receive an identification signal from the treatment tool identifier, the controller may restrict actuation of the treatment tool or the operation input unit.

Accordingly, this can prevent the operator from unintentionally actuating the treatment tool.

A second aspect of the present invention provides a medical system including the aforementioned medical apparatus; a medical device having an elongated insertion section, a bendable section provided at a distal end portion of the insertion section, and a driver that electrically drives the bendable section, the insertion section being provided with a channel into which the body section of the treatment tool is inserted; and a manipulation-target switching unit that switches a manipulation target to be manipulated with the operation input unit between the joint section of the treatment tool and the bendable section of the medical device. The controller controls the driver that corresponds to the manipulation target selected by the manipulation-target switching unit.

According to the present invention, by manipulating one of the treatment tool and the medical device selected by the manipulation-target switching unit, the operation input unit can be used to manipulate not only the joint section of the treatment tool but also the bendable section of the medical device.

In the second aspect, the manipulation-target switching unit may include a sensor that detects whether the manipulation target is one of the medical device and the treatment tool, and the controller may switch a target to be controlled by the controller to the manipulation target detected by the sensor.

Accordingly, when the operator changes the device to be used from the medical device to the treatment tool or from the treatment tool to the medical device, the sensor detects this change of the device to be used, and the manipulation target to be manipulated with the operation input unit can be automatically changed.

In the second aspect, in a case where the sensor does not detect either of the medical device and the treatment tool, the controller may restrict actuation of the medical device, the treatment tool, or the operation input unit.

Accordingly, this can prevent the medical device, the treatment tool, or the operation input unit from performing an operation unintended by the operator.

### {Advantageous Effects of Invention}

The present invention is advantageous in that it achieves high operability by allowing advancing-retracting and twisting operations of a treatment tool having joints and manipulation of the joints to be both performed with ease by using only one hand.

### {Brief Description of Drawings}

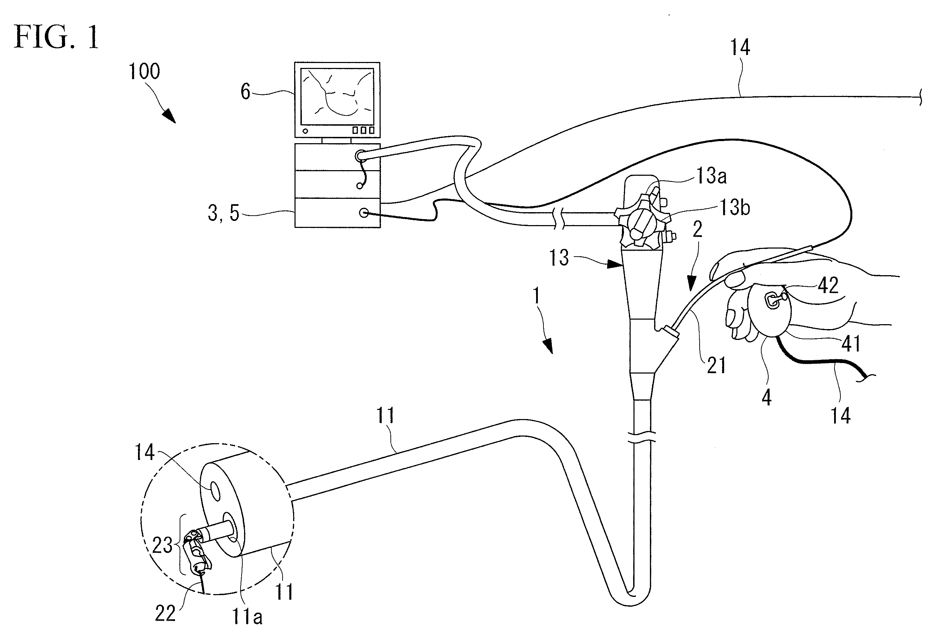

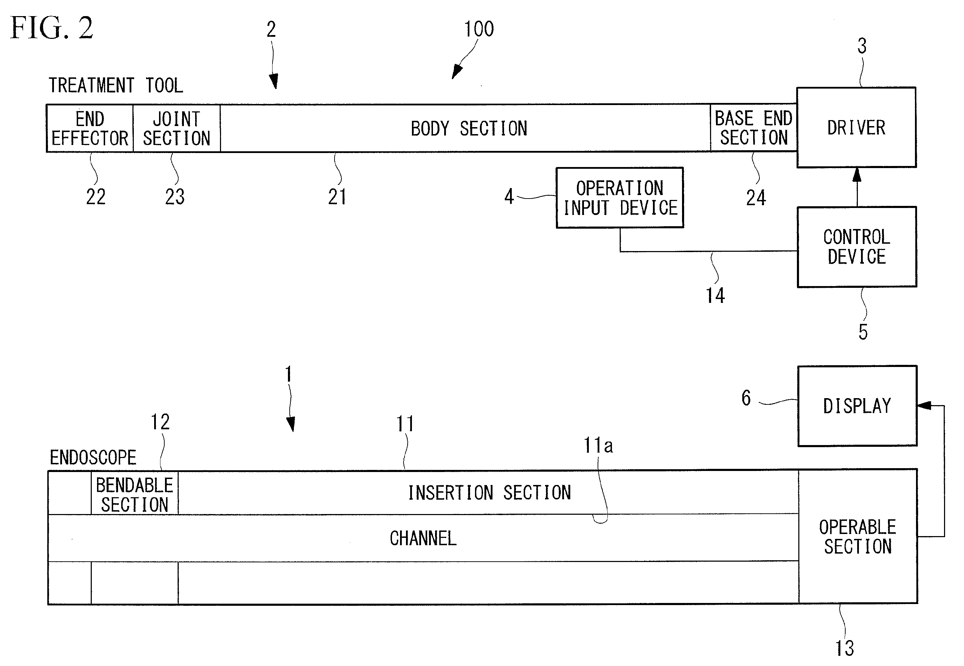

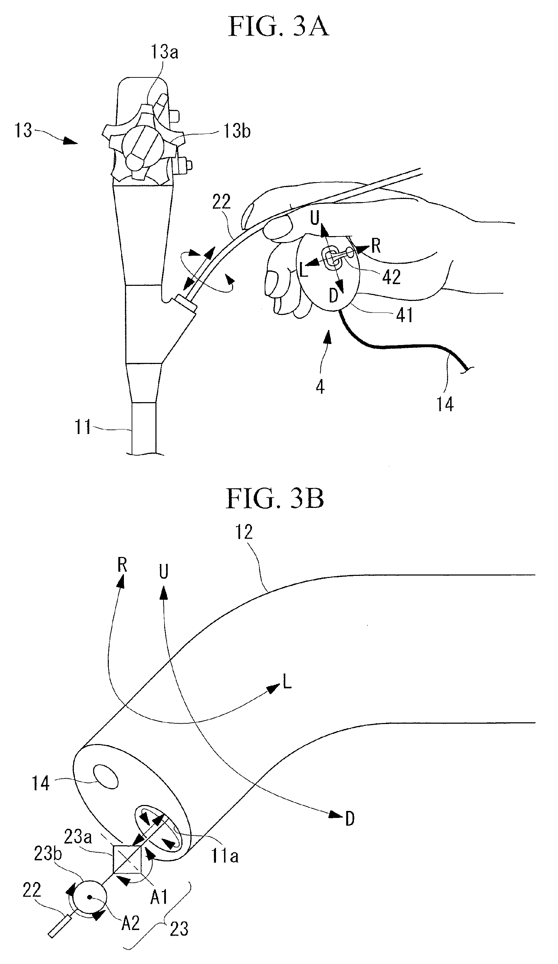

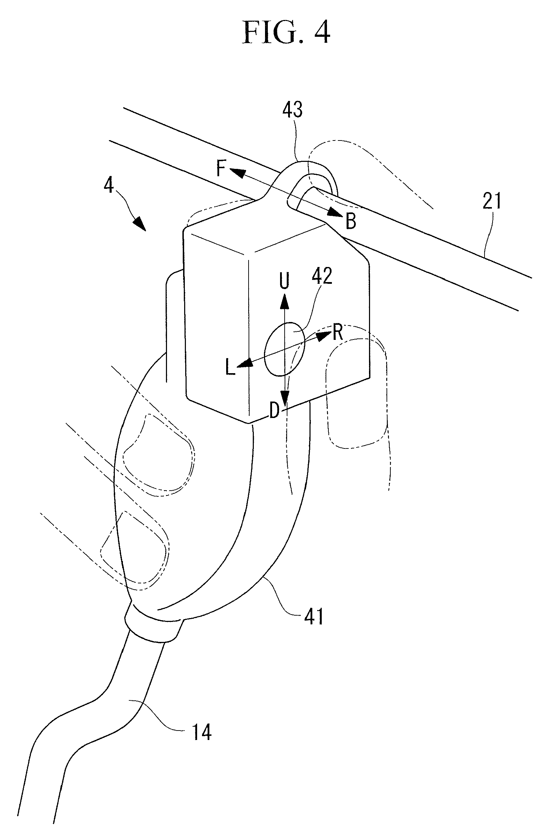

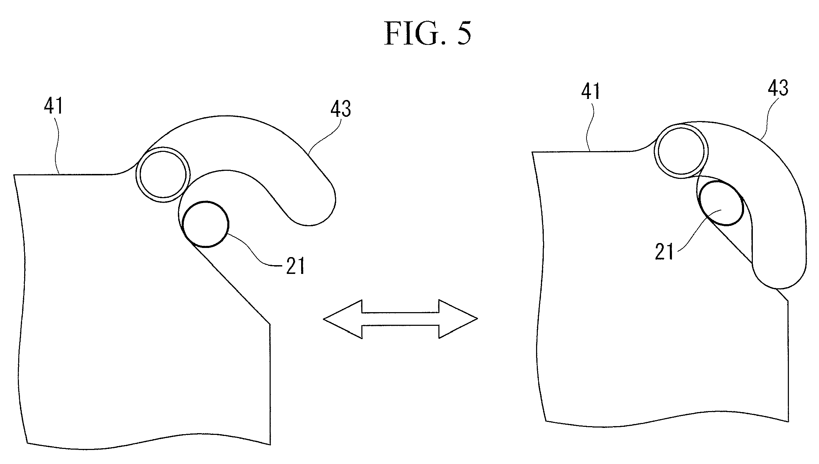

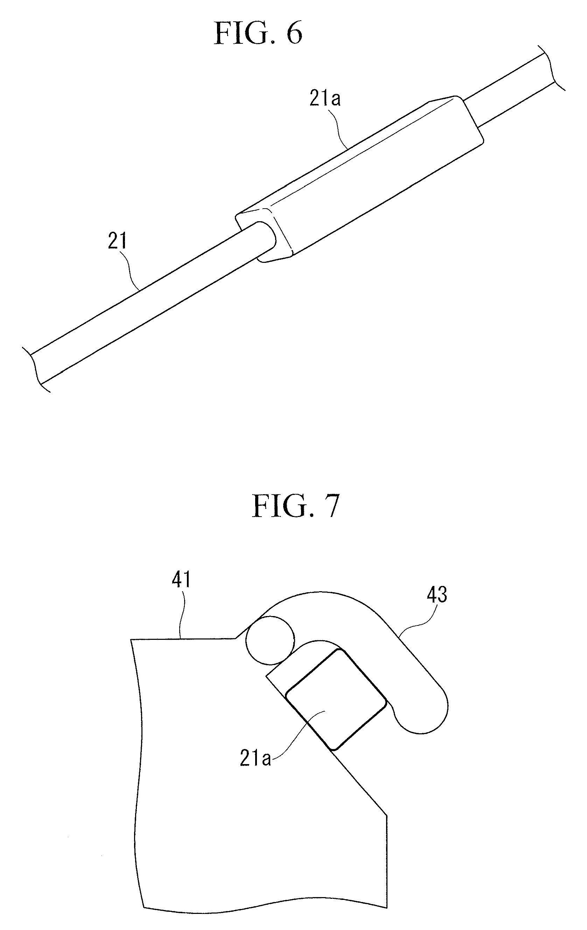

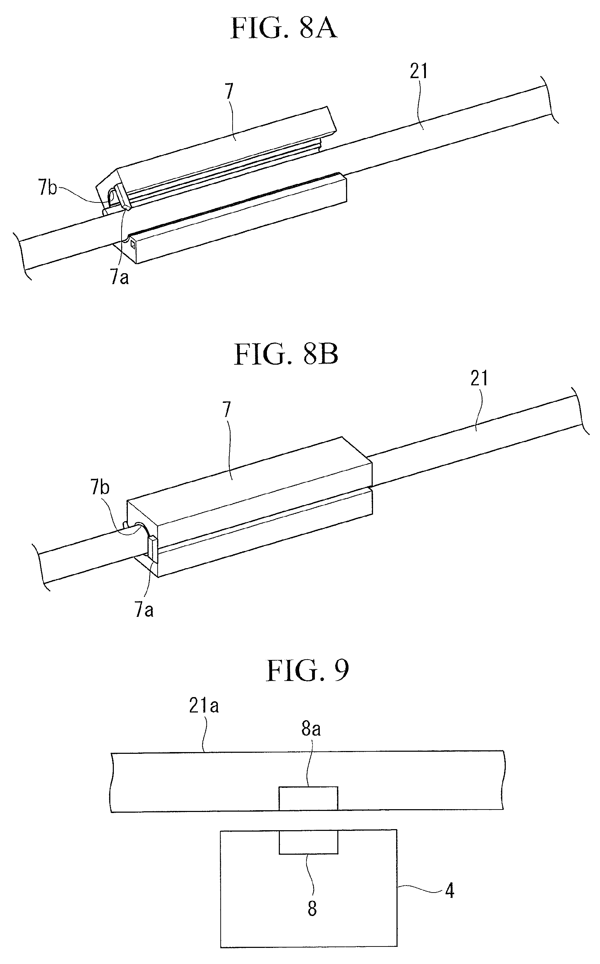

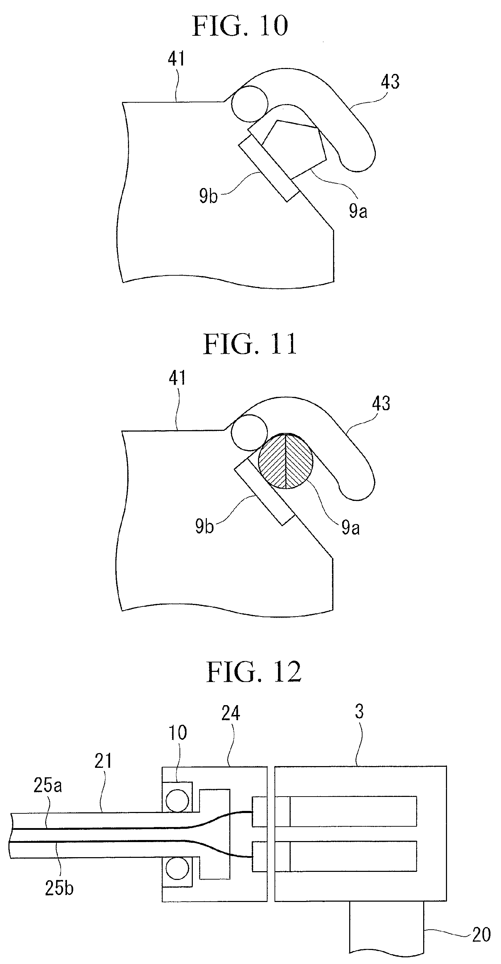

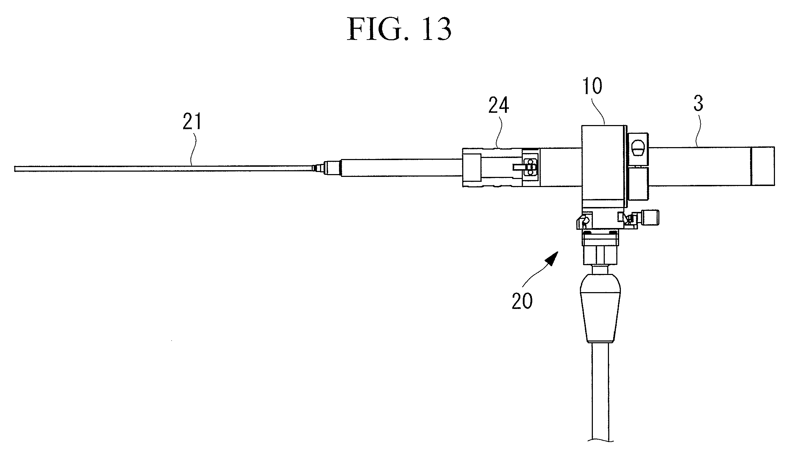

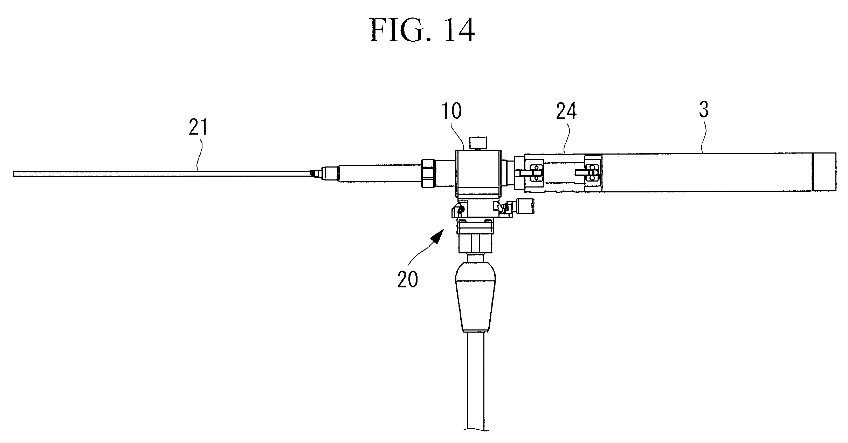

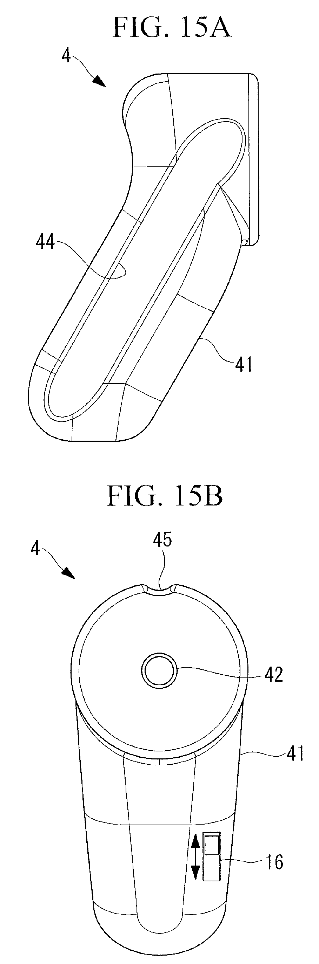

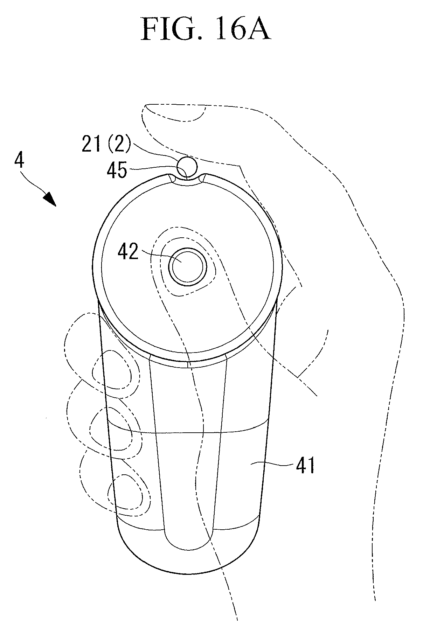

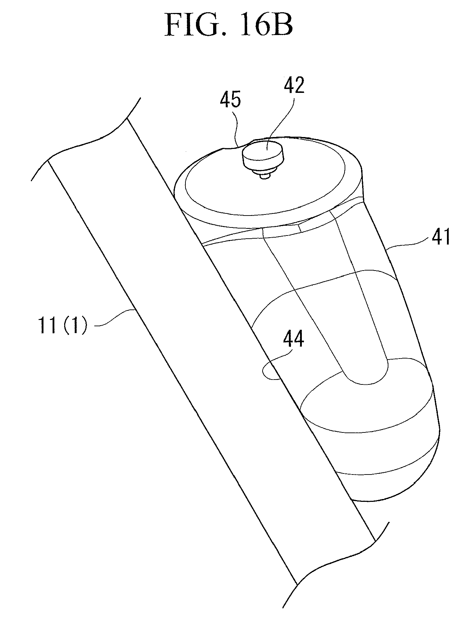

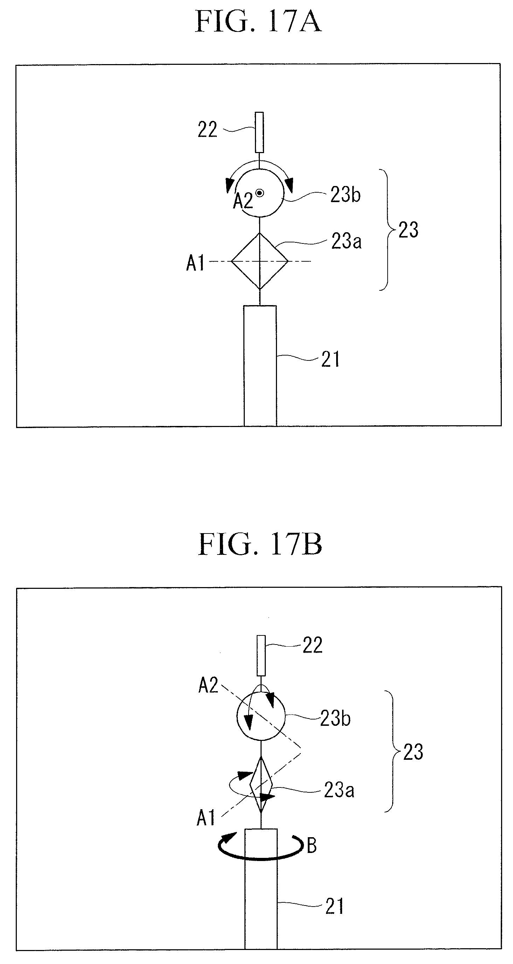

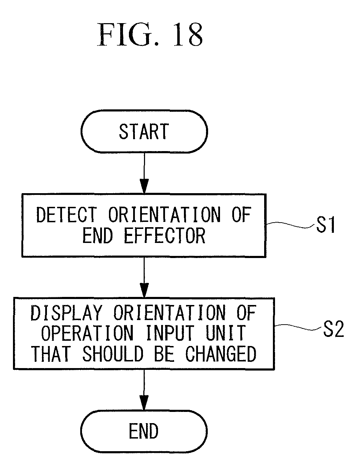

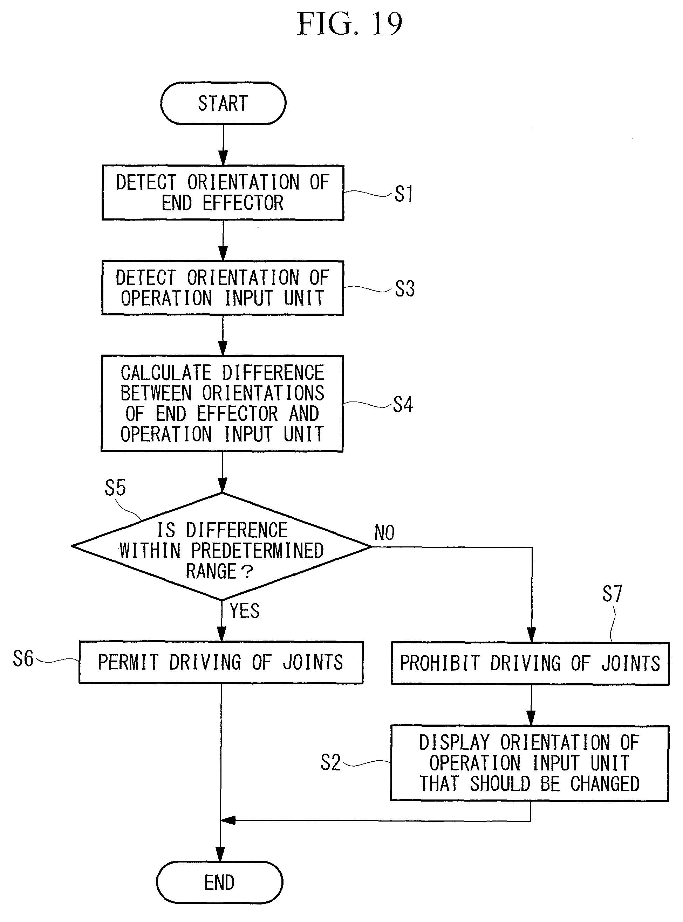

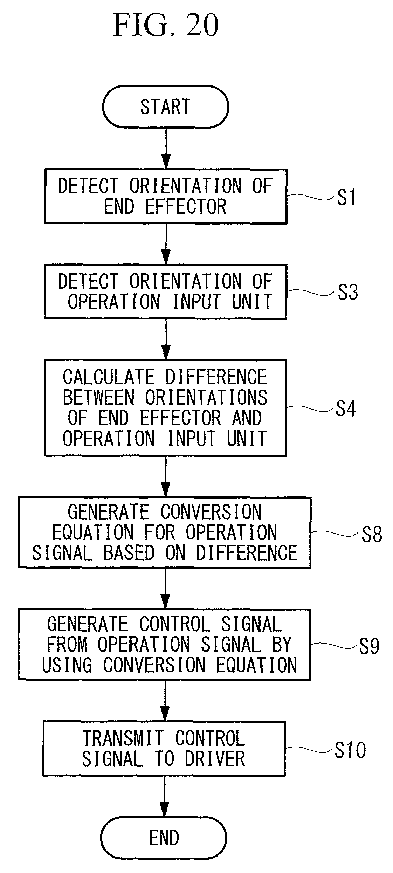

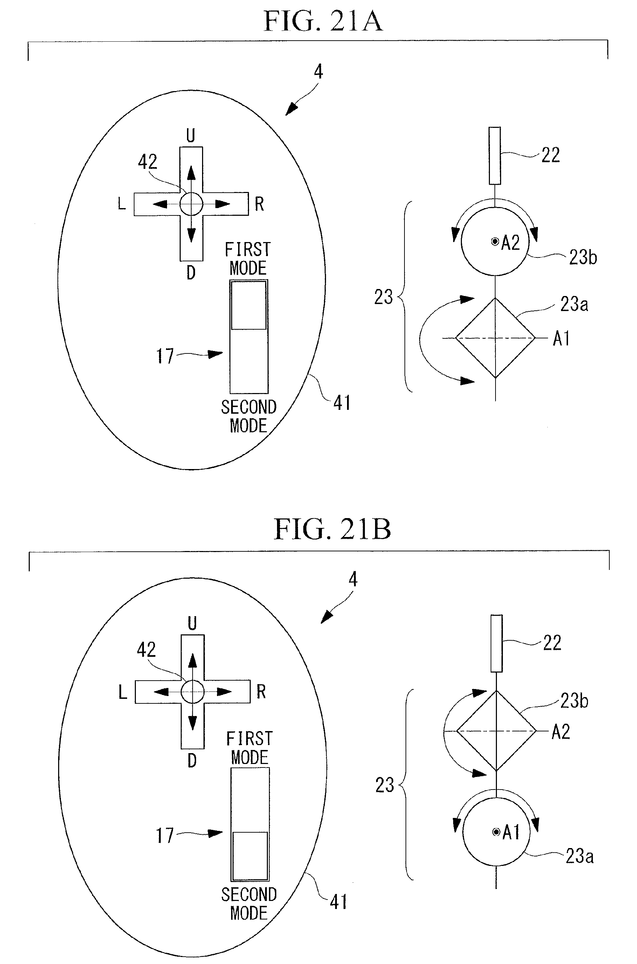

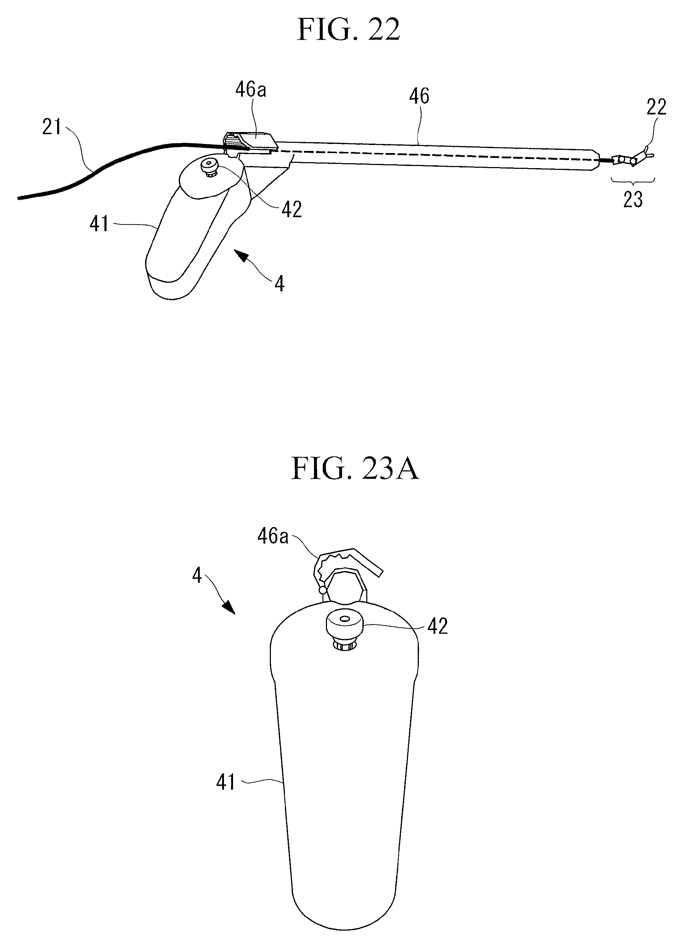

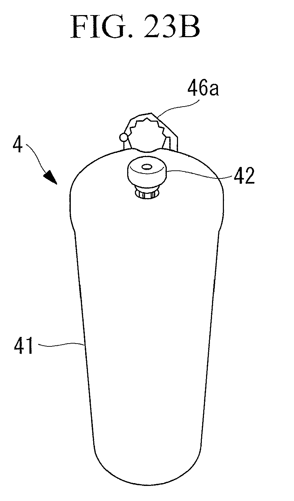

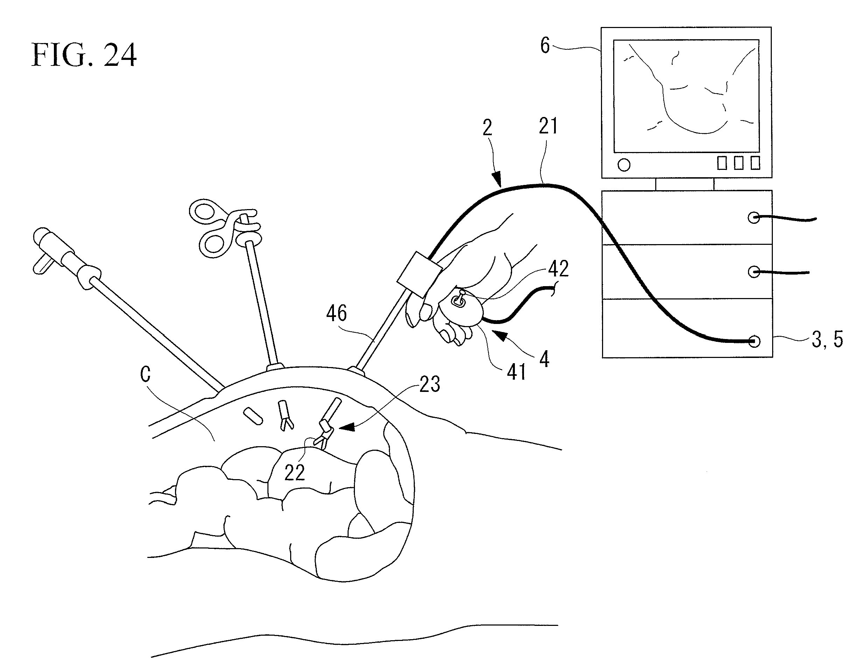

{Fig. 1} Fig. 1 is an external view illustrating the overall configuration of a medical system according to an embodiment of the present invention.
{Fig. 2} Fig. 2 is a block diagram illustrating the overall configuration of the medical system in Fig. 1.
{Fig. 3A} Fig. 3A illustrates how an endoscope and a treatment tool are manipulated.
{Fig. 3B} Fig. 3B illustrates the relationship between the manipulation of the endoscope and the treatment tool in Fig. 3A and the movement of an end effector.
{Fig. 4} Fig. 4 is a perspective view illustrating a modification of an operation input device equipped with a gripping member.
{Fig. 5} Fig. 5 illustrates an opening-closing operation of the gripping member.
{Fig. 6} Fig. 6 is a configuration diagram illustrating a modification of a body section equipped with a grip portion.
{Fig. 7} Fig. 7 illustrates a state where the grip portion in Fig. 6 is gripped by the gripping member.
{Fig. 8A} Fig. 8A is a configuration diagram illustrating an attachment member in an open state.
{Fig. 8B} Fig. 8B is a configuration diagram illustrating the attachment member in a closed state.
{Fig. 9} Fig. 9 is a schematic configuration diagram illustrating a modification of the operation input device equipped with a treatment tool identifier.
{Fig. 10} Fig. 10 illustrates a modification of the operation input device equipped with a direction recognizing mechanism.
{Fig. 11} Fig. 11 illustrates another modification of the operation input device equipped with the direction recognizing mechanism.
{Fig. 12} Fig. 12 is a configuration diagram illustrating an example of a rotatable support mechanism.
{Fig. 13} Fig. 13 is a configuration diagram illustrating another example of the rotatable support mechanism.
{Fig. 14} Fig. 14 is a configuration diagram illustrating another example of the rotatable support mechanism.
{Fig. 15A} Fig. 15A is a side view illustrating a modification of the operation input device having a groove for holding the endoscope.
{Fig. 15B} Fig. 15B is a front view of the operation input device in Fig. 15A.
{Fig. 16A} Fig. 16A illustrates a method of how the operation input device in Fig. 15A is used and shows a state where the operation input device is attached to the treatment tool.
{Fig. 16B} Fig. 16B illustrates the method of how the operation input device in Fig. 15A is used and shows a state where the operation input device is attached to the endoscope.
{Fig. 17A} Fig. 17A illustrates an endoscopic image when a distal-end treatment section is properly oriented in the circumferential direction.
{Fig. 17B} Fig. 17B illustrates an endoscopic image when the distal-end treatment section is improperly oriented in the circumferential direction.
{Fig. 18} Fig. 18 is a flowchart illustrating a method for correcting the correspondence relationship between an operational direction of the operation input device and a movement direction of the end effector.
{Fig. 19} Fig. 19 is a flowchart illustrating another method for correcting the correspondence relationship between the operational direction of the operation input device and the movement direction of the end effector.
{Fig. 20} Fig. 20 is a flowchart illustrating another method for correcting the correspondence relationship between the operational direction of the operation input device and the movement direction of the end effector.
{Fig. 21A} Fig. 21A is a configuration diagram illustrating a modification of the operation input device equipped with a correspondence changing unit and shows the relationship between the orientation of a joint section in the circumferential direction and a mode selected by the correspondence changing unit.
{Fig. 21B} Fig. 21B is a configuration diagram illustrating the modification of the operation input device equipped with the correspondence changing unit and shows the relationship between the orientation of the joint section in the circumferential direction and a mode selected by the correspondence changing unit.
{Fig. 22} Fig. 22 is a configuration diagram illustrating another modification of the operation input device to be attached to a treatment tool for laparoscopic surgery.
{Fig. 23A} Fig. 23A illustrates a state where the body section is released by a lock mechanism provided in a port member in Fig. 22.
{Fig. 23B} Fig. 23B illustrates a state where the body section is secured by the lock mechanism provided in the port member in Fig. 22.
{Fig. 24} Fig. 24 illustrates a method of how the operation input device in Fig. 22 is used in laparoscopic surgery.

### {Description of Embodiment}

A medical system 100 according to an embodiment of the present invention will be described below with reference to the drawings.

As shown in Figs. 1 and 2, the medical system 100 according to this embodiment includes a medical device 1 to be used by being inserted into the body, a treatment tool 2 to be inserted into a channel 11a provided in the medical device 1, a driver 3 that drives a joint section 23 of the treatment tool 2, an operation input device (operation input unit) 4 to which a command for manipulating the joint section 23 of the treatment tool 2 is input by an operator, and a control device (controller) 5.

A medical apparatus according to the present invention is constituted by the treatment tool 2, the driver 3, the operation input device 4, and the control device 5.

The medical device 1 is, for example, an endoscope, a shape lock member, or a manipulator. The medical device 1 has an elongated insertion section 11 to be inserted into the body of a patient and at least one channel 11a extending through the insertion section 11 in the longitudinal direction thereof. In this embodiment, it is assumed that the medical device 1 is an endoscope. The endoscope will be denoted by reference sign 1 hereinafter.

The endoscope 1 includes a bendable section 12 provided at the distal end portion of the insertion section 11 and an operable section 13 provided at the base end of the insertion section 11. The distal-end surface of the endoscope 1 is provided with a camera 14 that acquires, from above, a bird's-eye-view image of the treatment tool 2 protruding from an outlet of the channel 11a. Reference sign 6 denotes a display that displays an endoscopic image acquired by the camera 14. The bendable section 12 is bendable in four directions corresponding to up-down (UD) directions and left-right (LR) directions of the endoscopic image acquired by the camera 14. The operable section 13 is provided with two angle levers 13a and 13b that are manually operable by the operator. When the angle lever 13a is operated, the bendable section 12 bends in the UD directions. When the angle lever 13b is operated, the bendable section 12 bends in the LR directions.

The treatment tool 2 includes an elongated body section 21, an end effector (distal-end treatment section) 22 located at the distal end of the body section 21, the joint section 23 that couples the body section 21 and the end effector 22 to each other, and a base end section 24 provided at the base end of the body section 21. The treatment tool 2 is connected to the driver 3 at the base end section 24.

The body section 21 has an outside diameter that is smaller than the inside diameter of the channel 11a in the endoscope 1 and is advanceable-retractable and rotatable within the channel 11a.

The end effector 22 is, for example, forceps, scissors, or an electric scalpel for medically treating a treatment site.

The joint section 23 has, in the following order from the base end, a first joint 23a rotatable about a first axis A1 and a second joint 23b rotatable about a second axis A2. The first axis A1 and the second axis A2 are orthogonal to the longitudinal direction of the body section 21 and are also orthogonal to each other. Thus, the orientation of the end effector 22 in the endoscopic image can be changed in the UD directions by driving one of the first joint 23a and the second joint 23b and can also be changed in the LR directions by driving the other joint.

Although the rotational directions of the joints 23a and 23b are defined as the UD directions and the LR directions in this embodiment for illustrative purposes, the rotational directions of the joints 23a and 23b are not limited to these directions so long as the rotational directions are different from one another.

As shown in Figs. 3A and 3B, the end effector 22 has a total of six degrees of freedom of motion corresponding to the bending movement of the bendable section 12 in the UD and LR directions, the advancing-retracting movement and the rotational movement of the treatment tool 2, and the rotational movement of the joint section 23 in the UD and LR directions. Therefore, the end effector 22 can be disposed at any position and in any orientation within the movable range thereof.

The operation input device 4 includes a main body 41 having a shape and size that can be manually held by the operator with one hand and a joystick 42 (operable member) provided in the main body 41. Although Figs. 1, 3A, and 3B illustrate the main body 41 as having an elliptically spherical shape, the shape of the main body 41 is appropriately changeable. The joystick 42 is provided at a position where it is operable with the thumb of one hand holding the main body 41, and is operable in the UD directions and the LR directions. The operation input device 4 generates a UD operation signal and an LR operation signal on the basis of the amounts by which the joystick 42 is operated in the UD directions and the LR directions, respectively, and transmits the generated operation signals to the control device 5.

Furthermore, the operation input device 4 is provided separately from the treatment tool 2 and the driver 3 and can be carried independently of the treatment tool 2 and the driver 3.

In the reference drawings, the operation input device 4 and the control device 5 are connected to each other by a cable 14, and the operation signals are transmitted from the operation input device 4 to the control device 5 via the cable 14. Alternatively, the operation signals may be transmitted from the operation input device 4 to the control device 5 by wireless communication.

Furthermore, the type of operable member is not limited to the joystick 42 and may be, for example, four directional keys for the up, down, left, and right directions.

In the case where the operation input device 4 is connected to the control device 5 via the cable 14, the operation input device 4 is configured to be connectable to and disconnectable from the control device 5. Consequently, by detaching the operation input device 4 from the control device 5, the operation input device 4 alone can be used as an independent unit.

The body section 21, which directly comes into contact with the patient, and the operator's hands, which will touch the body section 21, are cleansed and sterilized before use and are maintained in a clean state during a medical operation. Because the operation input device 4 will directly come into contact with the body section 21 and the operator's hands, the operation input device 4 also needs to be sterilized at least before use. The operation input device 4, which does not contain components vulnerable to a sterilization process, such as electronic circuits and motors, is independently detachable from the other components so that the operation input device 4 can be sterilized by using a common sterilization method, such as autoclave sterilization or gamma-radiation sterilization.

Furthermore, since the operation input device 4 may become dirty during a medical operation, the operation input device 4 may be of a disposable type that is discarded after each usage. Even in that case, it is advantageous to make the operation input device 4 independently detachable from the other components.

The driver 3 electrically drives and rotates the first joint 23a and the second joint 23b. Specifically, the driver 3 generates a driving force in accordance with a drive signal received from the control device 5 and supplies the generated driving force to the base ends of drive wires (not shown) connected to the joints 23a and 23b. The drive wires extend from the joints 23a and 23b to the base end section 24 via the interior of the body section 21. The base ends of the drive wires are pushed and pulled in the longitudinal direction by the driving force so that the joints 23a and 23b are rotationally driven.

By using the UD operation signal and the LR operation signal received from the operation input device 4, the control device 5 generates, from the UD operation signal, a first drive signal for rotationally driving the first joint 23a and generates, from the LR operation signal, a second drive signal for rotationally driving the second joint 23b. Then, the control device 5 transmits the drive signals to the driver 3. The driver 3 is provided with a motor (not shown) in correspondence with each drive wire. The motors are actuated in accordance with the drive signals. Thus, the first joint 23a rotates when the operator operates the joystick 42 in the LR directions, and the second joint 23b rotates when the operator operates the joystick 42 in the LR directions.

Instead of driving the joints 23a and 23b in correspondence with the operational directions of the joystick 42, as described above, the controller 3 may drive the two joints 23a and 23b in a coordinated manner in response to up, down, left, and right inputs so as to move the end effector 22 in the up, down, left, and right directions in the coordinate system of the endoscopic image in accordance with the operation of the joystick 42.

The driver 3 and the control device 5 are disposed, for example, on the floor or on a rack, a table, or a surgical table placed on the floor. Although the driver 3 is shown as being contained in the control device 5 in Fig. 1, the driver 3 and the control device 5 may be separate units.

Next, the operation of the medical system 100 having the above-described configuration will be described.

In order to perform a medical treatment inside the body of the patient by using the medical system 100 according to this embodiment, the operator first inserts the insertion section 11 of the endoscope 1 into the body of the patient. Then, the operator inserts the body section 21 of the treatment tool 2 into the channel 11a and sets the distal end of the insertion section 11 and the end effector 22 at appropriate positions within the body.

Subsequently, in this example, the operator holds the operation input device 4 and the treatment tool 2 with his/her right hand. Specifically, the main body 41 is held with the palm, the middle finger, the ring finger, and the little finger of the right hand, and a part of the body section 21 extending outward from the channel 11a is held between the thumb and the index finger. Because the thumb is also used for operating the joystick 42, the body section 21 may be held by sandwiching the body section 21 between the index finger and the operation input device 4 and pressing the body section 21 against the operation input device 4 with the index finger. Moreover, the operator holds the operable section 13 of the endoscope 1 with his/her hand opposite the hand holding the operation input device 4 (i.e., the left hand in this example).

In this state, the operator operates the angle levers 13a and 13b with the fingers on his/her left hand so as to bend the bendable section 12 in the UD directions and the LR directions, whereby the entire end effector 22 can be moved in the UD directions or the LR directions. Furthermore, the operator operates the joystick 42 with the thumb on his/her right hand so as to rotate the first joint 23a and the second joint 23b, whereby the orientation of the end effector 22 can be changed in the UD directions and the LR directions. Moreover, the operator can make the end effector 22 advance and retract by causing the body section 21 to advance and retract in the longitudinal direction by using the thumb and the index finger on his/her right hand, and can also rotate the end effector 22 by twisting the body section 21.

In this case, the operator adjusts the orientation of the end effector 22 relative to the insertion section 11 so that the directions in which the end effector 22 is moved by the rotation of the second joint 23b correspond with the LR directions of the endoscopic image. Thus, the operational directions of the joystick 42 and the movement directions of the end effector 22 within the endoscopic image can be made to correspond with each other, and the end effector 22 can be intuitively manipulated.

In this case, according to this embodiment, since the operation input device 4 and the driver 3 are separate units, the operation input device 4 alone can be carried within the extendable range of the cable 14 in a state where the driver 3 is placed on the floor. In other words, because the weight acting on the right hand of the operator only includes the weight of the operation input device 4 and the cable 14, which are sufficiently light and do not include heavy components such as motors, the operator can continuously hold the operation input device 4 with ease, which is advantageous in that the level of fatigue of the operator can be reduced. Moreover, by using both hands, one operator can hold all of the operable section 13 of the endoscope 1, the body section 21 of the treatment tool 2, and the operation input device 4 with ease and can readily operate these three objects. Thus, one person can manipulate the end effector 22 in a total of six degrees of freedom of motion, which is advantageous in that a user-friendly medical system 100 can be achieved.

Furthermore, according to this embodiment, since the operation input device 4 can be carried independently of the driver 3 connected to the base end of the treatment tool 2, the operator can hold a freely-chosen position of the body section 21 with his/her right hand. In other words, this is advantageous in that the operator can hold the body section 21 near an inlet of the channel 11a and perform advancing-retracting and twisting operations on the body section 21 at this position so as to effectively cause the end effector 22 to advance or retract and to rotate.

Furthermore, when the operator twists the body section 21, the rotation amount of the end effector 22 may sometimes be smaller than the amount by which the body section 21 is rotated by the operator. This is because the rotational motion attenuates while being transmitted through the body section 21 due to the effect of, for example, friction acting on the body section 21 within the channel 11a. This causes a deviation to occur in the correspondence relationship between the direction in which the joystick 42 is operated by the operator and the movement direction of the end effector 22 within the endoscopic image. In this case, since the position and orientation of the operation input device 4 relative to the treatment tool 2 are freely changeable in this embodiment, the deviation in the aforementioned correspondence relationship can be readily corrected. Specifically, the operator first operates the joystick 42 in the LR directions so as to check the deviation angle between the coordinate system of the joystick 42 and the coordinate system of the end effector 22, and subsequently adjusts the angle of the operation input device 4 relative to the body section 21 by this deviation angle, thereby making the LR directions of the joystick 42 correspond with the movement directions of the second joint 23b.

In this embodiment, the operator holds the body section 21 with the thumb and the index finger of the hand that is holding the operation input device 4. Alternatively, as shown in Fig. 4, the operation input device 4 may include a gripping member 43 that sandwiches the body section 21 together with the main body 41 in the radial direction so as to grip the body section 21 by friction. In Fig. 4, the two-dot chain lines denote the operator's fingers holding the operation input device 4. Accordingly, the load on the index finger can be reduced as compared with a case where the body section 21 is directly held with the index finger, whereby the body section 21 can be held with more ease.

In this case, like the front-rear, up-down, and left-right movement directions of the end effector 22 within the endoscopic image, the longitudinal direction (BF direction) of the body section 21 gripped by the gripping member 43 is preferably orthogonal to the UD directions and the LR directions of the joystick 42.

Furthermore, as shown in Fig. 5, the gripping member 43 may be provided in a relatively movable manner between a position distant from the main body 41 (see left part of the drawing) and a position close to the main body 41 (see right part of the drawing).

Moreover, the cross-sectional shape of a part of the body section 21 that is located near the inlet of the channel 11a and that is located at the outer side of the channel 11a (this part will be referred to as "grip portion 21a" hereinafter) is preferably a shape other than a perfect circle, and is more preferably a polygonal shape, as shown in Fig. 6. Accordingly, when the operator directly holds the grip portion 21a with his/her fingers, the grip portion 21a can be held stably, so that rotational torque can be applied to the body section 21 more readily. Furthermore, in the case where the gripping member 43 is provided, the grip portion 21a can be gripped stably between the gripping member 43 and the main body 41, as shown in Fig. 7, so that the operator can apply rotational torque to the body section 21 even more readily.

Instead of modifying the grip portion 21a of the body section 21, a square-tube-like attachment member 7 may be attached to the side surface of the body section 21, as shown in Figs. 8A and 8B. The attachment member 7 is formed by connecting two semi-tubular parts, which are obtained by dividing a square tube member in the axial direction thereof, with a hinge so as to be openable and closable. Fig. 8A illustrates the open state, and Fig. 8B illustrates the closed state. Reference sign 7a denotes a lock mechanism that locks the opening motion of the attachment member 7 into a closed state. In the closed state, the attachment member 7 grips the grip portion 21a with friction between the inner surface thereof and the side surface of the body section 21. In order to increase the friction between the attachment member 7 and the body section 21, the inner surface of the attachment member 7 may be provided with an anti-slip member 7b composed of, for example, rubber.

The attachment member 7 preferably has rigidity higher than that of the body section 21. The body section 21 having low rigidity tends to buckle or elastically deform during advancing-retracting and twisting operations, and sometimes, the advancing-retracting and twisting operations performed by the operator are not transmitted to the distal end of the body section 21. By attaching the attachment member 7 having sufficient rigidity to the body section 21, the body section 21 can be prevented from buckling or elastically deforming, whereby the movement of the hand holding the body section 21 can be reliably transmitted to the distal end of the body section 21.

In this embodiment, the operation input device 4 may include a treatment tool identifier 8 that identifies the treatment tool 2 gripped by the gripping member 43, as shown in Fig. 9, and the control device 5 may generate a control signal for the treatment tool 2 identified by the treatment tool identifier 8.

Specifically, a medium 8a having stored therein identification information about the treatment tool 2 is attached to the grip portion 21a. The treatment tool identifier 8 is provided in the main body 41 of the operation input device 4 at a position that faces the gripping member 43. The medium 8a is, for example, an RFID tag or a bar code. The treatment tool identifier 8 has a reading means that is compliant with the type of the medium 8a. Thus, when the treatment tool 2 is to be used, the treatment tool identifier 8 in contact with the medium 8a reads the identification information from the medium 8a in a state where the grip portion 21a of the body section 21 is gripped by the gripping member 43. The treatment tool identifier 8 transmits a signal indicating the identified treatment tool 2 to the control device 5.

Furthermore, the treatment tool identifier 8 may repeatedly transmit the signal indicating the identified treatment tool 2 to the control device 5 at predetermined time intervals. In that case, if the control device 5 cannot receive the signal from the treatment tool identifier 8 at the predetermined time intervals, the control device 5 may determine that the treatment tool 2 is not gripped by the operation input device 4 and may limit the operation of the treatment tool 2 or limit input to the operation input device 4. This can prevent the operator from performing unintended operations.

Sometimes, multiple treatment tools 2 are simultaneously connected to the control device 5 via the driver 3 such that these multiple treatment tools 2 are set in a usable state at all times. In this case, the operator may select and use an arbitrary one of the treatment tools 2. In such a case, the function provided for identifying the treatment tool 2 gripped by the gripping member 43 allows the control device 5 to reliably and automatically identify the treatment tool 2 in use and to properly drive the joints 23a and 23b of that treatment tool 2.

In this embodiment, the operation input device 4 may include a direction recognizing mechanism that recognizes the orientation of the treatment tool 2 in the circumferential direction thereof. With the direction recognizing mechanism, the orientation of the end effector 22 protruding from the distal end of the insertion section 11 can be readily ascertained close-at-hand.

Figs. 10 and 11 illustrate an example of the direction recognizing mechanism. As shown in Figs. 10 and 11, the direction recognizing mechanism includes a recognition section 9a provided in the body section 21 and a detector 9b provided in the operation input device 4. The recognition section 9a is provided at the grip portion 21a and has properties that vary from position to position in the circumferential direction. The detector 9b is provided at a position facing the gripping member 43 and detects the properties of the recognition section 9a that is in contact with the detector 9b. Based on the properties detected by the detector 9b, the position of the treatment tool 2 in the circumferential direction can be recognized.

Specifically, as shown in Fig. 10, the recognition section 9a is constituted of the grip portion 21a having a polygonal cross-sectional shape, all sides of which have different lengths. Alternatively, as shown in Fig. 11, the recognition section 9a is constituted of the grip portion 21a having colors that vary from position to position in the circumferential direction. In Fig. 10, a contact sensor that detects contact with the recognition section 9a is used as the detector 9b. The orientation of the body section 21 in the circumferential direction is recognized based on the fact that the area of the side surface of the recognition section 9a in contact with the contact sensor varies depending on the orientation of the body section 21 in the circumferential direction. In Fig. 11, a colorimetric sensor that detects the color of the recognition section 9a is used as the detector 9b. The orientation of the body section 21 in the circumferential direction is recognized based on the fact that the color of the side surface of the recognition section 9a in contact with the colorimetric sensor varies depending on the orientation of the body section 21 in the circumferential direction.

The recognition section 9a and the detector 9b may also be used for identifying the aforementioned treatment tools 2. For example, the color of the recognition section 9a may be varied for each treatment tool 2. By using the colorimetric sensor for detecting the color of the recognition section 9a, the treatment tool 2 can be identified based on the detected color.

In this embodiment, a rotatable support mechanism 10 may be provided for supporting the base end section 24 of the treatment tool 2 in a rotatable manner in the circumferential direction, as shown in Figs. 12 to 14.

In a case where the base end section 24 is fixed to the driver 3, torsion occurs between the position of the body section 21 held by the operator and the base end section 24 when the operator twists the body section 21, causing the operator who is holding the body section 21 to feel a torque force occurring as a result of the torsion. Since the direction of this torque force is opposite to the direction in which the body section 21 is twisted by the operator, the operator feels a resistance force against the twisting operation. By providing the rotatable support mechanism 10, the torsion occurring in the body section 21 is cancelled out by the rotation of the base end section 24, so that the operator can twist the body section 21 with more ease without feeling a load.

In Fig. 12, the base end section 24 is rotatable due to the rotatable support mechanism 10, such as a ball bearing provided at the base end section 24 of the treatment tool 2. In Fig. 13, the driver 3 is rotatably supported by the rotatable support mechanism 10 provided at a supporter 20 that supports the driver 3. The driver 3 rotates due to torsion of the body section 21, thus also causing the base end section 24 to rotate. Reference signs 25a and 25b denote drive wires for driving the joints 23a and 23b. Alternatively, as shown in Fig. 14, the rotatable support mechanism 10 may be provided at the supporter 20 that supports the base end of the body section 21, and the body section 21 may be rotatably supported by the supporter 20. In Fig. 14, the driver 3 is not shown.

In this embodiment, the operation input device 4 may be provided with a groove 44 that can accommodate a part of the side surface of the insertion section 11 of the endoscope 1, as shown in Figs. 15A and 15B. In place of the gripping member 43, the operation input device 4 in this example has a groove 45 that can accommodate a part of the side surface of the body section 21. During a medical operation, the operator sometimes manually performs an advancing-retracting operation or a twisting operation also on the insertion section 11 of the endoscope 1. In this case, the operation input device 4 is removed from the treatment tool 2 and the insertion section 11 is fitted into the groove 44 and is held with the index finger, so that manual manipulation of the endoscope 1 and manipulation of the joint section 23 of the treatment tool 2 using the operation input device 4 can be performed concurrently.

In this case, a manipulation-target switching unit for switching a manipulation target to be manipulated with the operation input device 4 between the treatment tool 2 and the endoscope 1 may be provided. When the manipulation target is set to the endoscope 1 by the manipulation-target switching unit, the bendable section 12 may be bent in the up, down, left, or right direction by operating the joystick 42 in the up, down, left, or right direction. In this case, the bendable section 12 of the endoscope 1 is electrically drivable by a driver (not shown).

For example, as shown in Fig. 15B, a manipulation-target switching unit 16 is a toggle switch provided in the operation input device 4. The operator can use the toggle switch to select one of the treatment tool 2 and the endoscope 1 as the manipulation target. The operation input device 4 transmits, to the control device 5, a signal indicating the manipulation target selected by the toggle switch together with an operation signal. Based on these signals, the control device 5 transmits, to the driver 3 for the manipulation target selected by the operator, a control signal generated from the operation signal, and actuates the joint section 23 or the bendable section 12 serving as the manipulation target.

Alternatively, the manipulation-target switching unit may include a sensor that detects the presence or absence of the endoscope 1 or the treatment tool 2 within the groove 44 or 45, and may use this sensor to automatically recognize whether the operation input device 4 is attached to one of the endoscope 1 and the treatment tool 2. The sensor is, for example, a contact sensor, an infrared sensor, or a pushbutton switch provided at the inner surface of each of the grooves 44 and 45. If the sensor detects that there is no endoscope 1 or treatment tool 2 within the groove 44 or 45, the control device 5 may apply restrictions, such as stopping the operation of the endoscope 1 or the treatment tool 2 or disabling any operation by the operation input device 4. Accordingly, this can prevent the endoscope 1 or the treatment tool 2 from performing an operation unintended by the operator as a result of the operator performing an unintended operation on the operation input device 4. Furthermore, as shown in Figs. 16A and 16B, when the operator who is holding the endoscope 1 or the treatment tool 2 exchanges it for the other, the manipulation target to be manipulated with the operation input device 4 can be changed automatically. For example, in a case where the operator holding the treatment tool 2 exchanges it for the endoscope 1, the manipulation target to be manipulated with the operation input device 4 is changed to the bendable section 12 of the endoscope 1. In a case where the operator holding the endoscope 1 exchanges it for the treatment tool 2, the manipulation target to be manipulated with the operation input device 4 is changed to the joint section 23 of the treatment tool 2.

In this embodiment, the treatment tool 2 may be provided with a direction detector that detects the orientation of the end effector 22. If a deviation occurs in the correspondence relationship between the up, down, left, and right operational directions of the joystick 42 and the up, down, left, and right movement directions of the end effector 22 within the endoscopic image, an indication prompting the operator to correct this deviation may be displayed on the display 6. The direction sensor is, for example, a rotation-amount detector, such as an encoder, and detects the orientation of the end effector 22 in the circumferential direction thereof relative to the endoscope 1. Instead of using a direction sensor, the orientation of the end effector 22 may be detected from an image of the end effector 22 within the endoscopic image by using image recognition.

Specifically, as shown in Fig. 17A, the orientation of the end effector 22 when the first axis A1 is oriented in the LR directions of the endoscopic image is set as a reference. Then, as shown in Fig. 18, the orientation of the end effector 22 is detected by using the direction sensor (step S1), and an indication with regard to a deviation of the detected orientation of the end effector 22 from the reference, such as an arrow B shown in Fig. 17B, is displayed within the endoscopic image (step S2). The operator rotates the operation input device 4 in the direction indicated by this arrow B relative to the body section 21 so that the correspondence relationship between the operational directions of the joystick 42 and the movement directions of the joints 23a and 23b can be corrected.

A direction sensor may also be provided in the operation input device 4 in addition to the end effector 22. In this case, as shown in Fig. 19, the orientation of the operation input device 4 may further be detected by the direction sensor (step S3), and the difference between the two detected orientations of the end effector 22 and the operation input device 4 is calculated (step S4).

Then, if the difference is within a predetermined range (YES in step S5), the manipulation of the joints 23a and 23b using the operation input device 4 proceeds (step S6). In contrast, if the difference is outside the predetermined range (NO in step S5), the control device 5 forcedly discontinues the control of the joints 23a and 23b regardless of whether or not there is an operation signal from the operation input device 4 (step S7). Then, the aforementioned indication, for example, the arrow B, is displayed in the endoscopic image based on the difference between the two orientations (step S2).

Accordingly, the joints 23a and 23b can be prohibited from being manipulated in an inappropriate state where a deviation between the coordinate system of the end effector 22 and the coordinate system of the joystick 42 is sufficiently large.

In the case where the two direction sensors are provided, the driving of the joints 23a and 23b may proceed while compensating for a control signal based on the deviation between the two coordinate systems, as shown in Fig. 20, instead of prohibiting the manipulation of the joints 23a and 23b by the operator. Specifically, after calculating the difference between the two orientations of the end effector 22 and the operation input device 4 (step S4), a conversion equation for converting an operation signal into a control signal is generated based on the difference between these two orientations (step S8). By using the conversion equation, a control signal is generated from the operation signal (step S9). The control signal is then transmitted to the driver 3 (step S10).

As an alternative to the control shown in Figs. 18 to 20, the operation input device 4 may be provided with a correspondence changing unit 17, such as a toggle switch, which changes the correspondence relationship between the operational directions of the joystick 42 and the joints 23a and 23b, as shown in Figs. 21A and 21B.

As shown in Figs. 21A and 21B, when the orientation of the end effector 22 changes by 90 degrees, the rotational directions of the two joints 23a and 23b within the endoscopic image become inverted.

As shown in Fig. 21A, when a first mode is selected by the correspondence changing unit 17, an operation performed on the joystick 42 in the UD directions is set in correspondence with the first joint 23a, and an operation in the LR directions is set in correspondence with the second joint 23b. On the other hand, as shown in Fig. 21B, when a second mode is selected by the correspondence changing unit 17, an operation performed on the joystick 42 in the UD directions is set in correspondence with the second joint 23b, and an operation in the LR directions is set in correspondence with the first joint 23a. Accordingly, the operational directions of the joystick 42 and the movement directions of the end effector 22 in the endoscopic image can properly correspond with each other in accordance with the orientation of the end effector 22, thereby ensuring that the end effector 22 can be manipulated intuitively.

In this embodiment, the gripping member 43 of the operation input device 4 may be tubular so as to constitute a port member 46 to be inserted into the body of the patient from the body surface, as shown in Fig. 22. For example, as shown in Fig. 24, the port member 46 is to be used in laparoscopic surgery. By forming a small hole in the abdomen and setting the port member 46 in this hole, the treatment tool 2 can be guided to an abdominal cavity C through the port member 46. The body section 21 inserted in the port member 46 can be secured to the port member 46 by a lock mechanism 46a provided at the base end of the port member 46. Fig. 23A illustrates a state where the body section 21 is released by the lock mechanism 46a, and Fig. 23B illustrates a state where the body section 21 is secured by the lock mechanism 46a.

Accordingly, even in a case where the medical device 1 is of a tubular type integrated with the operation input device 4, since the operation input device 4 is a separate unit from the driver 3, the operator can manipulate the treatment tool 2 with ease without feeling the weight of the driver 3.

### {Reference Signs List}

- 1: medical device, endoscope
- 2: treatment tool
- 3: driver
- 4: operation input device (operation input unit)
- 5: control device (controller)
- 6: display
- 7: attachment member
- 7a: lock mechanism
- 7b: anti-slip member
- 8a: medium
- 9a: recognition section (direction recognizing mechanism)
- 9b: detector (direction recognizing mechanism)
- 10: rotatable support mechanism
- 11: insertion section
- 11a: channel
- 12: bendable section
- 13: operable section
- 13a, 13b: angle lever
- 14: camera
- 16: manipulation-target switching unit
- 17: correspondence changing unit
- 20: supporter
- 21: body section
- 21a: grip portion
- 22: end effector (distal-end treatment section)
- 23: joint section
- 23a, 23b: joint
- 24: base end section
- 25a, 25b: drive wire
- 41: main body
- 42: joystick (operable member)
- 43: gripping member
- 44, 45: groove
- 46: port member
- 100: medical system
- A1, A2: axis

## Claims

1. A medical apparatus comprising:
a treatment tool having an elongated body section that has flexibility, a distal-end treatment section that is located at a distal end of the body section, and a joint section that couples the body section and the distal-end treatment section to each other;
a driver that is connected to a base end of the body section and that supplies a driving force to the joint section for actuating the joint section;
an operation input unit having a main body and an operable member and being a separate unit from the treatment tool and the driver, the main body having a size and shape that can be held with one hand of an operator, the operable member being disposed at a position where the operable member is operable using a finger of the one hand holding the main body and receiving an operation command for manipulating the joint section; and
a controller that controls the driver in accordance with the operation command input to the operation input unit.

2. The medical apparatus according to Claim 1,
wherein the operation input unit is connectable to and disconnectable from the controller.

3. The medical apparatus according to Claim 1 or 2,
wherein the body section includes a grip portion having a non-circular cross-sectional shape and disposed in an area located outside a channel in a medical device in a state where the body section is inserted in the channel.

4. The medical apparatus according to any one of Claims 1 to 3,
wherein the operation input unit includes a gripping member that grips the body section by sandwiching the body section together with the main body in a radial direction of the body section.

5. The medical apparatus according to any one of Claims 1 to 4, further comprising:
a rotatable support mechanism that rotatably supports a base end portion of the body section.

6. The medical apparatus according to any one of Claims 1 to 5,
wherein the joint section includes a plurality of joints that are rotatable about axes in directions different from each other,
wherein the controller controls the driver such that each joint set in correspondence with a direction of the operation command input to the operable member is rotated in accordance with the direction of the operation command, and
wherein the medical apparatus further comprises a correspondence changing unit that changes a correspondence relationship in the controller between the direction of the operation command and each joint.

7. The medical apparatus according to any one of Claims 1 to 6,
wherein the operation input unit includes a treatment tool identifier that identifies the treatment tool serving as a manipulation target to be manipulated with the operation input unit, and
wherein the controller controls the driver in accordance with the treatment tool identified by the treatment tool identifier.

8. The medical apparatus according to Claim 7, comprising two or more of the treatment tools.

9. The medical apparatus according to Claim 7 or 8,
wherein in a case where the controller cannot receive an identification signal of the treatment tool from the treatment tool identifier, the controller restricts actuation of the treatment tool or the operation input unit.

10. A medical system comprising:
the medical apparatus according to any one of Claims 1 to 9;
a medical device having an elongated insertion section, a bendable section provided at a distal end portion of the insertion section, and a driver that electrically drives the bendable section, the insertion section being provided with a channel into which the body section of the treatment tool is inserted; and
a manipulation-target switching unit that switches a manipulation target to be manipulated with the operation input unit between the joint section of the treatment tool and the bendable section of the medical device,
wherein the controller controls the driver that corresponds to the manipulation target selected by the manipulation-target switching unit.

11. The medical system according to Claim 10,
wherein the manipulation-target switching unit includes a sensor that detects whether the manipulation target is one of the medical device and the treatment tool, and
wherein the controller switches a target to be controlled by the controller to the manipulation target detected by the sensor.

12. The medical system according to Claim 11,
wherein in a case where the sensor does not detect either of the medical device and the treatment tool, the controller restricts actuation of the medical device, the treatment tool, or the operation input unit.
